# EUROPEAN PATENT APPLICATION

(11) **EP 4 160 181 A1**
(43) Date of publication of application: **05.04.2023**
(21) Application number: 20938390.0
(22) Date of filing: 10.10.2020
(51) Int. Cl.: G01N 1/30

(54) **MULTIPLE-STAINING SECTION PREPARATION METHOD FOR CYTOPATHOLOGY SAMPLE**

(30) Priority: 28.05.2020 CN 202010468262
(71) Applicant: Wang, Daoxiang, Beijing 100050 (CN)
(72) Inventor: CHU, Wenjiang, Santa Monica California 90405 (US); WANG, Jian, Beijing 100050 (CN)
(74) Representative: Epping - Hermann - Fischer
(86) International application number: PCT/CN2020/120071
(87) International publication number: WO 2021/238015

(57) **Abstract**

The present invention relates to a method for multiple staining and slide preparation for a cytopathological sample. In particular, it relates to a multiple staining method for immunohistochemical staining and/or nucleic acid chromogenic in situ hybridization staining and conventional cytopathological staining a cell sample. The present invention also relates to an ex vivo cell obtained by said method, a pathological slide carrying said cell, and a kit for said method.

## Description

### Technical field

The present invention relates to a method for multiple staining and slide preparation in the field of cytopathology. This method can simultaneously display detailed morphological characteristics of cells and expression information of one or more specific biomarkers on cell samples. The staining results are suitable for observation under an ordinary optical microscope. Under the premise of meeting the pathological diagnosis requirements of pathologists, the expressions of one or more specific biomarkers on the same cell can be observed at the same time. The present invention also relates to the cells obtained by the method and the pathological slide carrying the cells.

### Background

Cytopathology studies the causes and pathogenesis of diseases, as well as the changes in the physiological functions of cells during the occurrence of diseases mainly based on abnormal conditions within cells, so as to propose the basis for diagnosis and prevention and treatment of diseases. Clinical samples cover exfoliative cytology, fine needle aspiration cytology, blood circulating tumor cells, and other cytology (cytology during surgery, bone marrow, peripheral blood cytology, AIDS cytology, etc.).

Early conventional staining methods for cytopathology include Papanicolaou staining, Wright-Giemsa staining and the like. At present, the most commonly used is Diff-Quik. The staining solution is prepared by the rapid staining method recommended by the World Health Organization (WHO). Similar to Wright-Giemsa, it is modified from the principle of Papanicolaou staining technology. The staining results can show the characteristics of cell membranes, such as cell membrane folds and bulges; cytoplasmic mucus, fat granules, neuroendocrine granules; characteristics of cell nuclei, including the shape and size of the nucleus, the number and shape of nucleoli, the number of chromosomes, and the texture of chromatin, and provide cell morphology diagnostic information for cytopathologists.

The workflow of cytopathology is to first make pathological smears of cell samples, perform routine staining, and then perform cytopathological morphological diagnosis. With the development of science and technology, cytopathologists may perform immunocytochemical staining or nucleic acid chromogenic in situ hybridization staining on protein or nucleic acid markers of cells to further help diagnosis, decide prognosis, and select therapies. However, current cytopathological samples cannot be further stained by cellular immunohistochemistry or nucleic acid chromogenic in situ hybridization after routine morphological staining. This clinical disadvantage is caused by the following reasons:
1) The dyes of conventional cytopathological staining methods will prevent the binding of the primary antibody or the primary probe to the relevant target. Therefore, these dyes need to be removed to better achieve the immunohistochemical dyes of biomarkers. 2) After removing the dyes, a higher concentration of the protein antibody or the nucleic acid probe is often needed for staining, which is likely to cause false positive and mislead clinical diagnosis, treatment and prognosis. 3) The cell smear on the slide is in an open environment during the staining process. During the staining process, the staining solution should be constantly replaced, washed, and then discarded. As the staining solution and washing solution are continuously replaced and discarded, the cell samples on the pathological slides will fall off and be lost, affecting the accuracy of the results. 4) Even if there is no need to remove conventional staining in some cases, the immunohistochemical staining or chromogenic in situ hybridization staining process can cause the conventional dye to fall off, resulting in poor observation of cell morphological characteristics under conventional staining. 5) During normal immunohistochemistry or nucleic acid chromogenic in situ hybridization staining, in order to improve the sensitivity of staining, the chemical staining process will be increased as much as possible, which will cause excessive accumulation of chromogenic products and cover cells, which will affect the observation of morphological diagnosis.

Although immunohistochemical and chromogenic in situ hybridization staining have become indispensable auxiliary staining methods in histopathology, because of the above technical obstacles, the application of immunohistochemiscal and chromogenic in situ hybridization staining in cytopathology is still unconventional and has no extensive clinical application. Therefore, a new staining method is needed in the art to overcome the above-mentioned shortcomings of the prior art.

### Summary of the invention

The present invention relates to a multiple staining method for cells in the field of cytopathology, which can simultaneously display detailed diagnosable morphological characteristics of cells and expression information of one or more specific biomarkers on the same sample cell. The staining results are suitable for observation under a common optical microscope. Under the premise of meeting the pathological diagnosis requirements of pathologists, the expressions of one or more specific biomarkers on the same cell can be observed at the same time. The staining process does not lose cells and is suitable for multiple staining of samples comprising a small number of cells.

The present invention solves the technical difficulties of conventional cytopathological staining and multiple staining of biomarkers. After immunohistochemical or nucleic acid chromogenic in situ hybridization staining is performed on a cell, the same cell is then subjected to cytopathological conventional staining. By those methods, it is possible to obtain diagnostic-level of cell morphological characteristics and information of various biomarkers on the same cell under an ordinary optical microscope. The staining results can show the morphological characteristics required for cytopathological diagnosis, including but not limited to: morphology of cell membrane, such as cell membrane folds and overall cell morphology and size; cytoplasmic mucus, fat granules, neuroendocrine granules; the characteristics of cell nuclei, including the shape and size of cell nuclei, the number and shape of nucleoli, and the texture of chromatin. This method clearly provides cytopathologists with basic information about cell morphological diagnosis, and combines the expression information of one or more biomarkers on the same cell, which helps improve the accuracy of cytopathological diagnosis.

The present invention also relates to the innovation of cell immunohistochemical and nucleic acid chromogenic in situ hybridization staining.

Conventional immunohistochemical staining is carried out using a pathological slide as a carrier, and the expression of a cell protein marker on the conventional pathological slide is achieved by an immunohistochemical method. A primary antibody and a secondary antibody labeled with a chromogenic enzyme, which can specifically recognize the primary antibody, are added to the sample on the pathological slide. Then the corresponding substrate of the chromogenic enzyme is added. By catalyzing the reaction with the chromogenic enzyme, the precipitate of the chromogenic product is finally produced, and the expression signal of a specific biomarker in the cell is shown on the pathological slide.

The expression of a nucleic acid marker of a cell on a conventional pathological slide is observed by the method of nucleic acid chromogenic in situ hybridization staining. A specific nucleic acid fragment is added to the sample on the pathological slide as the original nucleic acid probe. Then, a special reagent that can recognize the original probe labeled with a chromogenic enzyme is added. After that, the corresponding substrate of the chromogenic enzyme is added. By catalyzing the reaction with the chromogenic enzyme, the precipitate of the chromogenic product is finally produced, and the expression signal of a specific nucleic acid in the cell is shown on the pathological slide.

The biggest difference between the above two methods is that one uses an antibody and the other uses a nucleic acid probe for initial labeling, and the principle of the subsequent chromogenic reaction is the same. After the pathological slide is prepared, it can be observed under ordinary optical microscope to help pathologists make further diagnosis.

The existing immunohistochemical and chromogenic in situ hybridization staining techniques have the following disadvantages for cytopathological samples:
1) The core of a cytopathological sample is cells. Each cell is individually adsorbed or placed on a pathological slide by smear and other methods to form a so-called cell smear. The cell smear sample is in an open environment during the staining process. During the staining process, the staining solution must be constantly replaced, washed, and then discarded. With the continuous replacement and discarding of staining solution and washing solution, it is inevitable that some cell samples on the pathological slide will fall off and be lost, which leads to the reduction of the sensitivity and accuracy of the staining result, and is easy to produce false negative result, especially when the sample contains a small number of cells.
2) Conventional cytopathology immunohistochemical staining and nucleic acid chromogenic in situ hybridization staining take pathological slides as the carriers of cell samples, and require direct and close contact between the cell samples and pathological slides. There should be no gaps between them to make the cells fall off the slide. However, the staining solution cannot reach the position where the cells and the glass slide contact, which forms a staining blind area at the position where the cells and the glass slide contact. Therefore, the cells cannot be fully stained in all directions. It leaves dead corners of the staining, and affects the sensitivity and accuracy of the staining results.
3) The chromogenic reaction of immunohistochemical staining and chromogenic in situ hybridization staining is carried out on pathological glass slides. Optically visible color products are produced by the catalytic reactions, which are concentrated and precipitated near the chromogenic enzyme at the cellular level, and represent expression signals targeting the biomarkers. The precipitated chromogenic products are opaque. If the deposits are too much and too dense on the cells, the chromogenic products will cover the characteristics of cell morphology and affect the pathological diagnosis of cells when observed under an ordinary optical microscope.

In the staining process of immunohistochemistry and chromogenic in situ hybridization, the inventors adopt a new and improved staining method of cellular immunohistochemistry and nucleic acid chromogenic in situ hybridization.
1) Changed staining order and process: A method which first stains and then smears the cells is adopted. Traditional cellular immunohistochemical and nucleic acid chromogenic in situ hybridization staining use pathological slides as carriers. The cell samples are first smeared and fixed, and then the cells fixed on the pathological slides are stained.
   The present invention does not use pathological slides as carriers for staining. Instead, the sample cells are placed in a container and stained in the form of cell suspension. After the cells are stained, they are smeared and fixed, and observed under a microscope. The order in which the sample cells are fixed is opposite to conventional methods.
2) Improved solution exchange and washing methods to avoid the loss of cell samples. Conventional immunohistochemical staining or nucleic acid chromogenic in situ hybridization staining is performed on pathological slides. Cell smears are in an open environment during the staining process. During the staining process, the staining solution should be constantly replaced, washed, and then discarded. With the continuous replacement and discard of staining solution and washing solution, the cell samples on the pathological slides will fall off and be lost, which will inevitably affect the final diagnosis of the sample. In the present invention, the inventors place the cells in a container, and complete the incubation of the primary antibody or nucleic acid probe, and the chromogenic reaction and other staining processes in the cell suspension. During several times of changing the staining reagents and rinsing solutions, centrifugal precipitation of cell samples and other means are used to ensure that there is no target cell in the discarded supernatant of rinsing solution, which greatly reduces the loss of clinical cell samples during the staining process. After finishing all the staining, the stained cells are smeared on the pathological glass slides to ensure the stable and reliable results.
3) The changed staining environment avoids blind area of the staining on cells in traditional staining methods. During traditional immunohistochemical staining and nucleic acid chromogenic in situ hybridization staining, the staining is carried out on pathological slides. The staining solution cannot reach the position where the cells and the glass slide contact, which forms a staining blind area and dead corner.
   In order to allow the sample cells to be fully stained and avoid the blind area and dead corner on the cells, the inventors do not use pathological slides as carriers during the staining and chemical chromogenic process, and always allow the cells to be fully immersed in and contact different staining reagents. Therefore, there is no blind area and dead corner. The staining effect of cells and the sensitivity of staining are increased and the false negative rate of staining is reduced, which are obviously helpful.
4) The improved chemical chromogenic method makes the distribution of the chromogenic products uniform. Conventional immunohistochemical staining and nucleic acid chromogenic in situ hybridization are carried out on pathological slides. The chromogenic reaction is carried out on fixed tissues. The chromogenic precipitates produced by catalysis are easy to accumulate locally. Because they are opaque and can cover the morphological characteristics of the microstructure of cells, they affect the morphological diagnosis of cytopathology. During the chromogenic process, the inventors constantly shake the cells suspended in the solution to uniformly disperse the chromogenic precipitates produced by the catalysis, so that they do not accumulate and affect the clearness of the optical path. The chromogenic products produced by the catalysis can be reasonably distributed on the cells, which can display the expression information of the biomarkers without hindering the diagnosis of cell morphology, and greatly improve the accuracy of the pathologist's diagnosis of cell morphology.
5) Improved and more effective method of eliminating background noise of staining. Conventional immunohistochemical staining and nucleic acid chromogenic in situ hybridization are performed on pathological slides, and the chromogenic reaction is performed on fixed tissues. Various staining reagents will non-specifically precipitate and adsorb on pathological slides or cell tissues. The position where the cell and the slide contact can also produce the so-called edge effect during the staining process, causing non-specific adsorption and precipitation of the staining components. It is necessary to wash the pathological slides many times to eliminate the non-specific adsorption and precipitation of reagents, and also increase the possibility of cell shedding and loss from the pathological slides.

After the staining reaction of the present invention is completed, individual cells are fully washed in the cell suspension without blind area and dead corner. At the same time, because there are no undesirable effects such as non-specific deposition and edge effects on pathological slides, it can greatly reduce non-specific background staining noise on the pathological slides. It is obviously helpful to improve the staining effect of cells, increase the sensitivity of staining, and reduce the false positive rate of staining results.

Specifically, the present invention relates to the following technical solutions:
In one aspect, the present invention relates to a multiple staining method for making immunohistochemical staining and/or nucleic acid chromogenic in situ hybridization staining and cell morphological staining that can be used for pathological diagnosis on a cell sample, comprising:
a) preparing cell suspension of the cell sample in a container;
b) performing immunohistochemical staining and/or nucleic acid chromogenic in situ hybridization staining on cells in the cell suspension in the container, comprising:
   i) adding a primary antibody and/or a primary nucleic acid probe that specifically binds to an antigen and/or a nucleic acid marker on the cells to the cell suspension to complete specific binding of the primary antibody and/or the primary nucleic acid probe to the antigen and/or the nucleic acid marker;
   ii) removing the unbound primary antibody and/or primary nucleic acid probe, and then preparing cell suspension again,
   iii) adding a secondary antibody conjugated with a chromogenic enzyme and/or a secondary nucleic acid probe labeled with a chromogenic enzyme to the cell suspension to specifically bind to the primary antibody and/or the primary nucleic acid probe;
   iv) removing the unbound secondary antibody conjugated with the chromogenic enzyme and/or secondary nucleic acid probe labeled with the chromogenic enzyme, and then preparing cell suspension again,
   v) adding a chromogenic substrate to the cell suspension for color development; during the color development process, constantly shaking the cells suspended in the cell suspension to uniformly disperse the chromogenic precipitates produced by catalysis to avoid concentrated accumulation; and
   vi) removing excess chromogenic substrate and then preparing cell suspension again; and
c) performing cell morphological staining that can be used for pathological diagnosis on the cells in the container, and then smearing the multiply stained cells on a pathological slide; or, smearing the immunohistochemically stained and/or nucleic acid chromogenic in situ hybridization stained cells on a pathological slide to perform cell morphological staining that can be used for pathological diagnosis.

In some embodiments, step a) comprises centrifuging and precipitating the cell sample added with a cell fixing solution, then preparing cell suspension in the container, and then adding a reagent that increases the permeability of cell membrane, which is conducive to staining of nucleus and cytoplasm.

In some embodiments, in steps ii), iv) and vi), removing the unbound primary antibody and/or the primary nucleic acid probe, removing the unbound secondary antibody conjugated with the chromogenic enzyme and/or secondary nucleic acid probe labeled with the chromogenic enzyme and removing the excess chromogenic substrate are carried out by the following steps:
i) washing the cells;
ii) centrifuging the precipitated cells; and
iii) discarding the supernatant.

In some embodiments, the cell morphological staining that can be used for pathological diagnosis is selected from Diff-Quik staining, Papanicolaou staining, Wright-Giemsa staining, and Hematoxylin/Eosin (H&E) staining, or a derivative or modified form thereof.

In some embodiments, the cell morphological staining that can be used for pathological diagnosis is Diff-Quik staining.

In some embodiments, the cell sample is selected from a cytopathological fine needle aspiration (FNA) sample, a tumor cell sample, a cervical scrape cell sample, and a urine exfoliated cell sample, or the cell sample is obtained by diluting a cell precipitate of a sample from a patient selected from body fluid, blood, serum, plasma, urine, saliva, sweat, sputum, semen, mucus, tear, lymph, amniotic fluid, interstitial fluid, pleural fluid, ascites, lung lavage fluid, cerebrospinal fluid, feces and a tissue sample.

In some embodiments, the primary antibody and/or primary probe is a combination of multiple antibodies and/or probes, which respectively stain cell membrane, cytoplasm, and/or cell nucleus.

In another aspect, the present invention relates to a multiply stained ex vivo cell, which has both morphological staining that can be used for pathological diagnosis and cell biomarker staining.

In some embodiments, the cell is derived from a cell sample selected from the group consisting of a fine needle aspiration (FNA) sample, a blood circulating tumor cell sample, a cervical scrape cell sample, a urine exfoliated cell sample, body fluid, blood, serum, plasma, urine, saliva, sweat, sputum, semen, mucus, tear, lymph, amniotic fluid, interstitial fluid, pleural fluid, ascites, lung lavage fluid, cerebrospinal fluid, feces and a tissue sample.

In some embodiments, the cell is a tumor cell, which has both morphological staining that can be used for pathological diagnosis and tumor marker staining.

In some embodiments, the cell is obtained by the method of the invention.

In another aspect, the present invention relates to a pathological slide, which carries the above-mentioned multiply stained ex vivo cells.

In another aspect, the present invention also relates to a multiple staining kit for making immunohistochemical staining and/or nucleic acid chromogenic in situ hybridization staining and cell morphological staining that can be used for pathological diagnosis on a cell sample. The kit comprises a reagent for immunohistochemical staining and/or nucleic acid chromogenic in situ hybridization staining on cell samples, and a reagent for cell morphological staining.

In some embodiments, the reagent for immunohistochemical staining and/or nucleic acid chromogenic in situ hybridization staining of a cell sample comprises a primary antibody and/or a primary nucleic acid probe, a secondary antibody conjugated with a chromogenic enzyme and/or a secondary nucleic acid probe labeled with a chromogenic enzyme, and a chromogenic substrate.

In some embodiments, the reagent used for cell morphological staining is selected from a reagent used for Diff-Quik staining, Papanicolaou staining, Wright-Giemsa staining, and H&E staining, or a derivative or modified form thereof.

### Description of the drawings

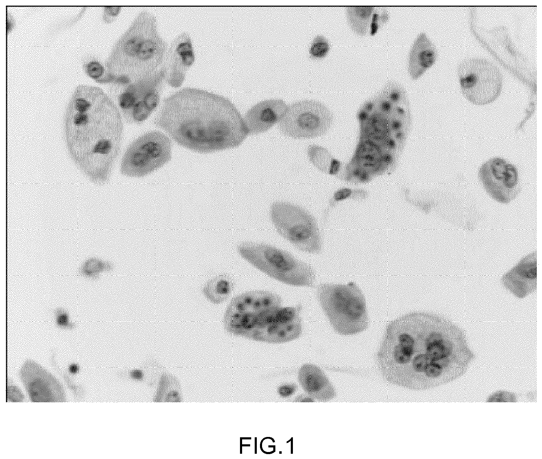

Figure 1 shows exfoliated urinary epithelial cells in urine, which are simultaneously multiply stained by Diff-Quik and liquid-phase immunohistochemistry. All exfoliated urinary epithelial cells are stained blue by Diff-Quik, which meets the requirements of pathologists for cell morphological diagnosis. Two of the urinary epithelial cells with atypical morphology are stained brown by liquid immunohistochemistry. The brown signal shown in the figure is the expression of cytokeratin 20 (CK20) in two exfoliated urinary epithelial cells.

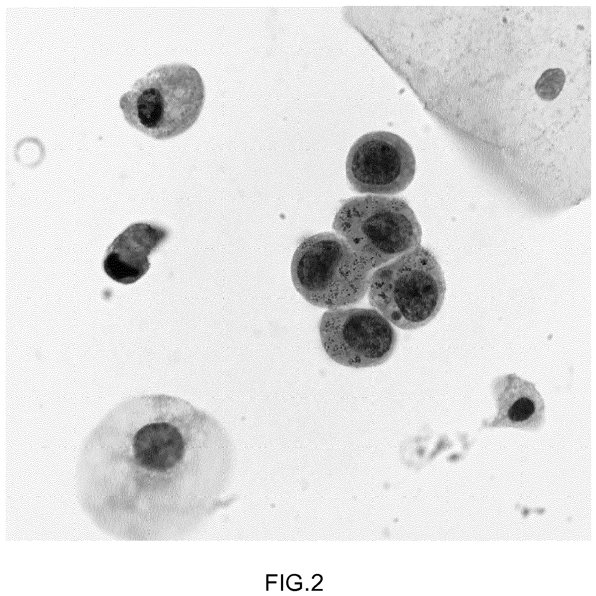

Figure 2 shows the exfoliated urinary epithelial cells in urine, which are simultaneously stained by Diff-Quik and liquid-phase nucleic acid chromogenic in situ hybridization. All exfoliated urinary epithelial cells are stained blue by Diff-Quik, which meets the requirements of pathologists for cell morphological diagnosis. Four of the cells that are morphologically diagnosed as urinary epithelial carcinoma are stained brown by nucleic acid chromogenic in situ hybridization. The brown signal shown in the figure corresponds to the expression of human transcription factor GATA-3 in exfoliated urothelial cancer cells.

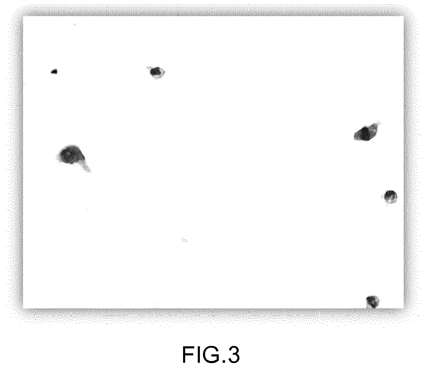

Figure 3 shows that the cells were immunohistochemically stained, smeared, and then were Diff-Quik stained. Two tumor cells in the field were stained brown. Three normal exfoliated epithelial cells in the urine were stained dark blue and pale pink by Diff-Quik. All cell stains can be used for cytopathological diagnosis.

### Detailed description

### Example 1

### Multiple staining of cells based on a new and improved cell immunochemical protein marker staining method and Diff-Quik

(All centrifugations were performed at 1500 r/min for 5 minutes. The supernatant was removed, and the pellet was then resuspended in PBS).
1. 25 ml of urine from bladder cancer patients containing 4% paraformaldehyde cell fixing solution was centrifuged to pellet the cells. The supernatant was discarded. The pellet was then diluted and resuspended in 1 ml of PBS (pH 7.4) buffer, and placed into 10 ml plastic test tube.
2. 20 microliters of Triton-100 was added (to increase permeability of cell membrane and facilitate staining of cytoplasm and nucleus).
3. The cell suspension was heated at 92 degrees Celsius for 5 minutes for antigen retrieval.
4. 100 microliters of Dual Endogenous Enzyme Block (Dako, Carpinteria, Ca) was added in the suspension, which was incubated for 5 minutes to inhibit the activities of endogenous peroxidase and alkaline phosphatase in the cells.
5. After centrifugation, the supernatant and the excess Dual Endogenous Enzyme Block was removed. The pellet was then diluted and resuspended in 1 ml of PBS (pH 7.4) buffer.
6. 200 microliters of Serum-Free Block (Dako) was added and was incubated for 5 minutes (to fill in non-specific protein binding sites).
7. The primary antibody, i.e., 200 microliters of CK20 mouse antibody was added, and was incubated for 2 hours at room temperature.
8. 1 ml of PBS buffer was added and mixed well. After centrifugation, the supernatant was removed. The step was repeated one to three times to wash away the unbound primary antibody.
9. 200 microliters of goat anti-mouse, polymerized peroxidase-conjugated secondary antibody (EnVision, Dako) was added and incubated at room temperature for 30 minutes.
10. The cell sample was washed three times with 1 ml of PBS buffer by centrifugation to remove the unbound secondary antibody.
11. A chromogenic reagent, peroxidase-Liquid DAB+ (from Dako) was added, and the cell suspension sample was placed on a shaker, and incubated for 10 minutes at the shaking speed of 600-2500 rpm.
12. 1 ml of PBS buffer was added, and the suspension was centrifuged to pellet the cells and to remove excess chromogenic reagent.
13. The stained cell sample was washed and rinsed with 1 ml PBS buffer. Then the suspension was centrifuged to pellet the cells, and the supernatant was discarded. The step was repeated three times to effectively eliminate non-specific staining background noise.
14. 1 ml of Diff-Quik Stain No. 2 (from Fisher Scientific, Cat. #22750012) was added. The suspension was incubated for 1 minute and then was centrifuged to remove excess chromogenic reagent;
15. 1 ml of Diff-Quik Stain No. 1 (from Fisher Scientific, Cat. #22750012) was added. The suspension was incubated for 1 minute and then was centrifuged to remove excess chromogenic reagent.
16. 200 µl PBS buffer was added, and the suspension was smeared on a slide.

The cytopathological smear was observed under a microscope, as shown in Figure 1.

The Diff-Quik staining may also be completed in the form of a pathological slide after the completion of immunohistochemical staining.

### Example 2

Multiple staining of cells based on a new and improved nucleic acid chromogenic in situ hybridization staining method and Diff-Quik

(All centrifugations were performed at 1500 r/min for 5 minutes. The supernatant was removed, and the pellet was then resuspended in PBS).
1. 25 ml of urine from bladder cancer patients containing 4% paraformaldehyde cell fixing solution was centrifuged to pellet the cells. The supernatant was discarded. The pellet was then diluted and resuspended in 1 ml of PBS (pH 7.4) buffer, and placed into 10 ml plastic test tube.
2. 20 microliters of Triton-100 was added (to increase permeability of cell membrane and facilitate staining of cytoplasm and nucleus).
3. RNAscope^{®} 2.5 HD Reagent Kit (ACD Bio, California, USA) immunohistochemical nucleic acid in situ hybridization kit was used to perform nucleic acid marker staining according to the protocol recommended by the manufacturer. RNA nucleic acid probes directed to human transcription factor GATA-3 (Hs-GATA3, ACD Bio, California, USA) were added, and the suspension was incubated at 40 degrees Celsius for 2 hours.
4. The cell suspension was centrifuged and the supernatant was discarded. After adding 1 ml of the washing buffer provided by the manufacturer, the cell suspension was centrifuged for 5 minutes and the supernatant was discarded. The washing buffer was added to repeat the washing once.
5. 4 drops of hybridization solution AMP1 was added, and the suspension was incubated at 40 degrees Celsius for 30 minutes.
6. The cell suspension was centrifuged and the supernatant was discarded. After adding 1 ml of the washing buffer, the cell suspension was centrifuged for 5 minutes, and the supernatant was discarded. Then the washing was repeated once.
7. 4 drops of hybridization solution AMP2 was added, and the suspension was incubated at 40 degrees Celsius for 30 minutes.
8. The cell suspension was centrifuged and the supernatant was discarded. After adding 1 ml of the washing buffer, the cell suspension was centrifuged for 5 minutes, and the supernatant was discarded. Then the washing was repeated once.
9. 4 drops of hybridization solution AMP3 was added, and the suspension was incubated at 40 degrees Celsius for 30 minutes.
10. The cell suspension was centrifuged and the supernatant was discarded. After adding 1 ml of the washing buffer, the cell suspension was centrifuged for 5 minutes, and the supernatant was discarded. Then the washing was repeated once.
11. 4 drops of hybridization solution AMP4 was added, and the suspension was incubated at 40 degrees Celsius for 15 minutes.
12. The cell suspension was centrifuged and the supernatant was discarded. After adding 1 ml of the washing buffer, the cell suspension was centrifuged for 5 minutes, and the supernatant was discarded. Then the washing was repeated once.
13. 4 drops of hybridization solution AMP5 was added, and the suspension was incubated at 40 degrees Celsius for 30 minutes.
14. The cell suspension was centrifuged and the supernatant was discarded. After adding 1 ml of the washing buffer, the cell suspension was centrifuged for 5 minutes, and the supernatant was discarded. Then the washing was repeated once.
15. 4 drops of hybridization solution AMP6 was added, and the suspension was incubated at 40 degrees Celsius for 15 minutes.
16. The cell suspension was centrifuged and the supernatant was discarded. After adding 1 ml of the washing buffer, the cell suspension was centrifuged for 5 minutes, and the supernatant was discarded. Then the washing was repeated once.
17. 120 microliters of pre-mixed DAB-A and DAB-B chromogenic solutions were added, and the cell suspension sample was placed on a shaker, and incubated for 10 minutes at the shaking speed of 600-2500 rpm. The cell suspension was centrifuged, and the supernatant was discarded.
18. The stained cell sample was washed and rinsed with 1 X rinsing solution. Then the suspension was centrifuged to pellet the cells, and the supernatant was discarded. The step was repeated three times to effectively eliminate non-specific staining background noise.
19. 1 ml of Diff-Quik Stain No. 2 (from Fisher Scientific, Cat. #22750012) was added. The suspension was incubated for 1 minute and then was centrifuged to remove excess chromogenic reagent.
20. 1 ml of Diff-Quik Stain No. 1 (from Fisher Scientific, Cat. #22750012) was added. The suspension was incubated for 1 minute and then was centrifuged to remove excess chromogenic reagent.
21. 200 µl PBS buffer was added, and the suspension was smeared on a slide. The cytopathological smear was observed under a microscope, as shown in Figure 2.
22. The Diff-Quik staining may also be completed in the form of a pathological slide after the completion of nucleic acid chromogenic in situ hybridization.

### Example 3

### Multiple staining of cells based on a new and improved cell immunochemical protein marker staining method and Diff-Quik

(All centrifugations were performed at 1500 r/min for 5 minutes. The supernatant was removed, and the pellet was then resuspended in PBS).
1. 25 ml of urine from bladder cancer patients containing 4% paraformaldehyde cell fixing solution was centrifuged to pellet the cells. The supernatant was discarded. The pellet was then diluted and resuspended in 1 ml of PBS (pH 7.4) buffer, and placed into 10 ml plastic test tube.
2. 20 microliters of Tween 20 was added (to increase permeability of cell membrane and facilitate staining of cytoplasm and nucleus).
3. The cell suspension was heated at 92 degrees Celsius for 5 minutes for antigen retrieval.
4. 100 microliters of Dual Endogenous Enzyme Block (Dako, Carpinteria, Ca) was added in the suspension, which was incubated for 5 minutes to inhibit the activities of endogenous peroxidase and alkaline phosphatase in the cells.
5. After centrifugation, the supernatant and the excess Dual Endogenous Enzyme Block was removed. The pellet was then diluted and resuspended in 1 ml of PBS (pH 7.4) buffer.
6. 200 microliters of Serum-Free Block (Dako) was added and was incubated for 5 minutes (to fill in non-specific protein binding sites).
7. The primary antibody, i.e., 200 microliters of CK20 mouse antibody was added, and was incubated for 2 hours at room temperature.
8. 1 ml of PBS buffer was added and mixed well. After centrifugation, the supernatant was removed. The step was repeated once to wash away the unbound primary antibody.
9. 200 microliters of goat anti-mouse, polymerized peroxidase-conjugated secondary antibody (EnVision, Dako) was added and incubated at room temperature for 30 minutes.
10. The cell sample was washed three times with 1 ml of PBS buffer by centrifugation to remove the unbound secondary antibody.
11. A chromogenic reagent, peroxidase-Liquid DAB+ (from Dako) was added, and the cell suspension sample was placed on a shaker, and incubated for 5 minutes at the shaking speed of 600-2500 rpm.
12. 1 ml of PBS buffer was added, and the suspension was centrifuged to pellet the cells and to remove excess chromogenic reagent.
13. The stained cell sample was washed and rinsed with 1 ml PBS buffer. Then the suspension was centrifuged to pellet the cells, and the supernatant was discarded. The step was repeated three times to effectively eliminate non-specific staining background noise. 200 µl of cell suspension was re-prepared.
14. The above cell suspension was used to prepare an ultra-thin Pap smear (ThinPrep) manually. That is, the cells were transferred and adsorbed on a pathological slide.
15. 1 ml of Diff-Quik Stain No. 2 (from Fisher Scientific, Cat. #22750012), which was diluted with PSB buffer to 5%, was added. The suspension was incubated for 30 seconds and then washed with 1ml of PBS buffer to remove excess chromogenic reagents;
16. 1 ml of Diff-Quik Stain No. 1 (from Fisher Scientific, Cat. #22750012), which was diluted with PSB buffer to 5%, was added. The suspension was incubated for 30 seconds and then then washed with 1ml of PBS buffer to remove excess chromogenic reagents.
17. The slide was covered and mounted.

The cytopathological smear was observed under a microscope, as shown in Figure 3.

## Claims

1. A multiple staining method for making immunohistochemical staining and/or nucleic acid chromogenic in situ hybridization staining and cell morphological staining that can be used for pathological diagnosis of on a cell sample, comprising:
a) preparing cell suspension of the cell sample in a container;
b) performing immunohistochemical staining and/or nucleic acid chromogenic in situ hybridization staining on cells in the cell suspension in the container, comprising:
i) adding a primary antibody and/or a primary nucleic acid probe that specifically binds to an antigen and/or a nucleic acid marker on the cells to the cell suspension to complete specific binding of the primary antibody and/or the primary nucleic acid probe to the antigen and/or the nucleic acid marker;
ii) removing the unbound primary antibody and/or primary nucleic acid probe, and then preparing cell suspension again,
iii) adding a secondary antibody conjugated with a chromogenic enzyme and/or a secondary nucleic acid probe labeled with a chromogenic enzyme to the cell suspension to specifically bind to the primary antibody and/or the primary nucleic acid probe;
iv) removing the unbound secondary antibody conjugated with the chromogenic enzyme and/or secondary nucleic acid probe labeled with the chromogenic enzyme, and then preparing cell suspension again,
v) adding a chromogenic substrate to the cell suspension for color development; during the color development process, constantly shaking the cells suspended in the suspension to uniformly disperse the chromogenic precipitates produced by catalysis to avoid concentrated accumulation; and
vi) removing excess chromogenic substrate and then preparing cell suspension again; and
c) performing cell morphological staining that can be used for pathological diagnosis on the cells in the container, and then smearing the multiply stained cells on a pathological slide; or, smearing the immunohistochemically stained and/or nucleic acid chromogenic in situ hybridization stained cells on a pathological slide to perform cell morphological staining that can be used for pathological diagnosis.

2. The method of claim 1, wherein in steps ii), iv) and vi), removing the unbound primary antibody and/or the primary nucleic acid probe, removing the unbound secondary antibody conjugated with the chromogenic enzyme and/or secondary nucleic acid probe labeled with the chromogenic enzyme, and removing the excess chromogenic substrate are carried out by the following steps:
i) washing the cells;
ii) centrifuging the precipitated cells; and
iii) discarding the supernatant.

3. The method of claim 1 or 2, wherein the cell morphological staining that can be used for pathological diagnosis is selected from Diff-Quik staining, Papanicolaou staining, Wright-Giemsa staining, and Hematoxylin/Eosin (H&E) staining, or a derivative or modified form thereof.

4. The method of claim 3, wherein the cell morphological staining that can be used for pathological diagnosis is Diff-Quik staining.

5. The method of any one of claims 1 to 3, wherein the cell sample is selected from a fine needle aspiration (FNA) sample, a blood circulating tumor cell sample, a cervical scrape cell sample, and a urine exfoliated cell sample, or the cell sample is obtained by diluting a cell precipitate of a sample from a patient selected from body fluid, blood, serum, plasma, urine, saliva, sweat, sputum, semen, mucus, tear, lymph, amniotic fluid, interstitial fluid, pleural fluid, ascites, lung lavage fluid, cerebrospinal fluid, feces and a tissue sample.

6. The method of any one of claims 1 to 5, wherein the primary antibody and/or primary probe is a combination of multiple antibodies and/or probes, which respectively stain cell membrane, cytoplasm, and/or cell nucleus.

7. A multiply stained ex vivo cell, which has both morphological staining that can be used for pathological diagnosis and cell biomarker staining.

8. The ex vivo cell of claim 7, wherein the cell is derived from a cell sample selected from the group consisting of a cytopathological fine needle aspiration (FNA) sample, a blood circulating tumor cell sample, a cervical scrape cell sample, a urine exfoliated cell sample, body fluid, blood, serum, plasma, urine, saliva, sweat, sputum, semen, mucus, tear, lymph, amniotic fluid, interstitial fluid, pleural fluid, ascites, lung lavage fluid, cerebrospinal fluid, feces and a tissue sample.

9. The ex vivo cell of claim 7, wherein the cell is a tumor cell, which has both morphological staining that can be used for pathological diagnosis and tumor marker staining.

10. The ex vivo cell of any one of claims 7 to 10, wherein the cell is obtained by the method of any one of claims 1 to 5.

11. A pathological slide, which carries the multiply stained ex vivo cell of any one of claims 7 to 10.

12. A multiple staining kit for making immunohistochemical staining and/or nucleic acid chromogenic in situ hybridization staining and cell morphological staining that can be used for pathological diagnosis of on a cell sample, which comprises a reagent for immunohistochemical staining and/or nucleic acid chromogenic in situ hybridization staining on cell samples, and a reagent for cell morphological staining.

13. The kit of claim 12, wherein the reagent for immunohistochemical staining and/or nucleic acid chromogenic in situ hybridization staining of a cell sample comprises a primary antibody and/or a primary nucleic acid probe, a secondary antibody conjugated with a chromogenic enzyme and/or a secondary nucleic acid probe labeled with a chromogenic enzyme, and a chromogenic substrate.

14. The kit of claim 12 or 13, wherein the reagent used for cell morphological staining is selected from a reagent used for Diff-Quik staining, Papanicolaou staining, Wright-Giemsa staining, and H&E staining, or a derivative or modified form thereof.

15. The kit of claim 13, wherein the primary antibody and/or primary probe is a combination of multiple antibodies and/or probes, which respectively stain cell membrane, cytoplasm, and/or cell nucleus.

16. The kit of any one of claims 12 to 15, wherein the cell sample is selected from a cytopathological fine needle aspiration (FNA) sample, a tumor cell sample, a cervical scrape cell sample, and a urine exfoliated cell sample, or the cell sample is obtained by diluting a cell precipitate of a sample from a patient selected from body fluid, blood, serum, plasma, urine, saliva, sweat, sputum, semen, mucus, tear, lymph, amniotic fluid, interstitial fluid, pleural fluid, ascites, lung lavage fluid, cerebrospinal fluid, feces and a tissue sample.
